# EUROPEAN PATENT APPLICATION

(11) **EP 4 524 239 A1**
(43) Date of publication of application: **19.03.2025**
(21) Application number: 23803594.3
(22) Date of filing: 11.05.2023
(51) Int. Cl.: C12N 5/0775, A61K 35/12, C12N 15/09, C12Q 1/02

(54) **METHOD FOR PRODUCING EXTRACELLULAR VESICLES CONTAINING BIOACTIVE SUBSTANCE**

(30) Priority: 12.05.2022 JP 2022078750
(71) Applicant: The University of Tokyo, Bunkyo-ku, Tokyo 113-8654 (JP)
(72) Inventor: TANAKA Yosuke, Tokyo 113-8654 (JP); WANG Shuo, Tokyo 113-8654 (JP)
(74) Representative: Vossius & Partner Patentanwälte Rechtsanwälte mbB
(86) International application number: PCT/JP2023/017656
(87) International publication number: WO 2023/219119

(57) **Abstract**

It is an object of the present invention to provide a method for efficiently producing extracellular vesicles containing biologically active materials such as sonic hedgehog (Shh). The present invention provides, as a solution, a method for producing extracellular vesicles, comprising obtaining extracellular vesicles secreted from cells subjected to a treatment of inactivating Rab GTPase such as Rab18, wherein the cells are preferably cells expressing Shh. In addition, the present invention provides, as a solution, an extracellular vesicle containing Shh and having a particle diameter of 800 nm or more and/or a volume of 10⁹ nm³ or more.

## Description

### Technical Field

The present invention relates to a method for efficiently producing extracellular vesicles containing biologically active materials.

### Background Art

Extracellular vesicles transport biologically active materials such as morphogens, lipids and miRNAs, and release these biologically active materials to the outside of cells. The released biologically active materials act on the recipient cells and alter their functions. Hsp90, a component of extracellular vesicles, promotes production of extracellular vesicles and bioactivity thereof through membrane deformation, which is essential for the release of extracellular vesicles. Neutral sphingomyelinase (nSMase) produces ceramide and promotes secretion of extracellular vesicles from multivesicular bodies (MVBs).

In addition, it has been reported that extracellular vesicles are also associated with wound healing, cancer promotion and progression, the pathological process and therapeutic course of diseases such as cardiovascular diseases and neurological diseases. Therefore, it is extremely important to understand the physiological functions of extracellular vesicles more deeply and to find a method for efficiently producing extracellular vesicles that show therapeutic effects on the diseases, in order to establish a novel therapeutic method for refractory diseases.

It has been found that extracellular vesicles (hMSC-EVs) derived from human mesenchymal stem cells (hMSCs) have the effect of promoting angiogenesis of human umbilical vein endothelial cells (HUVECs) and are considered to be potentially useful for recovery from myocardial infarction, atherosclerotic occlusive disease and surgical wounds (Non Patent Literature 1). Since hMSC-EVs have lower immunogenicity than hMSCs, administration of the hMSC-EVs as a therapeutic agent to patients is considered to be safer than direct administration of hMSCs. However, hMSC-EVs released from hMSCs cannot exert their full therapeutic effects if a pre-treatment has not been performed on the hMSCs to enhance the sonic hedgehog (Shh) signaling pathway (Non Patent Literature 2 to Non Patent Literature 4). For example, it has been reported that the pre-treatment of CD34⁺ hMSCs with Shh increases the secretion of Shh-containing extracellular vesicles (Shh-EVs), which is effective for the improvement of cardiovascular diseases including post-myocardial infarction syndrome (Non Patent Literatures 5 and 6).

The hedgehog signaling pathway plays an important role for individual cells to obtain correct positional information in embryonic development and tissue regeneration, and the hedgehog signaling pathway is a signaling pathway having a hedgehog protein as a center. Inhibition of this pathway causes severe developmental abnormalities and may even result in death (Non Patent Literature 7). It has been reported that, in adult bodies, abnormal hedgehog signals are involved in formation and progression of tumors such as osteoblastoma, basal cell carcinoma and rhabdomyosarcoma (Non Patent Literature 8 and Non Patent Literature 9, etc.). For this reason, research on the hedgehog pathway as a target for drug discovery has been progressing, and agonists, for example, are attracting attention for their utilization as therapeutic agents for cerebral ischemia such as cerebral infarction and cerebral hemorrhage (Non Patent Literature 10).

With regard to the hedgehog protein as a main role of the hedgehog pathway, three hedgehog homologs, namely, a sonic hedgehog, an Indian hedgehog, and a desert hedgehog have been identified in mammals. Among these, the sonic hedgehog has been the most studied. The sonic hedgehog functions as a morphogen, for example, in the determination of the body axis of the neural tube during early brain development.

Rab, a small molecular GTPase, regulates intracellular vesicular transport by binding its GTP-bound active form to various effectors (Non Patent Literature 11). It has been reported that there are many isoforms of Rab, and that Rab18 is one of the isoforms, which is contained in the Golgi apparatus, endoplasmic reticulum, lipid droplets, etc., as well as in Shh-EVs called ART-EVs (Non Patent Literature 12; Coulter et al., Cell reports 24, 973-986.e978, 2018). However, the role of Rab18 in Shh-EVs has not yet been understood well.

### Citation List

### Non Patent Literature

Non Patent Literature 1: Li et al., Adv Skin Wound Care 18, 491-500; quiz 501-492, 2005
Non Patent Literature 2: Mackie et al., Circ Res 111, 312-321, 2012
Non Patent Literature 3: Hyun et al., Sci Rep 5, 14135, 2015
Non Patent Literature 4: Jia et al., Acta neurochirurgica 163, 2297-2306, 2021
Non Patent Literature 5: Salybekov et al., International journal of molecular sciences 19, 3040, 2018
Non Patent Literature 6: Fleury et al., Front Immunol 5, 370, 2014
Non Patent Literature 7: Zhang et al., Cell 106, 781-792, 2001
Non Patent Literature 8: Sekulic and Von Hoff, Cell 164, 831, 2016
Non Patent Literature 9: Rimkus et al., Cancers 8, 22 2016 Doi:10.3390/cancers8020022
Non Patent Literature 10: Hadden, ChemMedChem 9, 27-37, 2014
Non Patent Literature 11: Dejgaard et al., Cellular and molecular life sciences : CMLS 76, 1935-1945, 2019
Non Patent Literature 12: Coulter et al., Cell reports 24, 973-986.e978, 2018

### Summary of Invention

### Technical Problem

In view of the above-described circumstances, it is an object of the present invention to provide a method for efficiently producing extracellular vesicles containing biologically active materials. More specifically, it is an object of the present invention to provide a method for efficiently producing extracellular vesicles containing sonic hedgehog (Shh), and novel extracellular vesicles characterized by containing a large amount of Shh.

### Solution to Problem

The present inventors have performed a treatment of inactivating Rab18 in mesenchymal stem cells. As a result, the present inventors have found that the content of sonic hedgehog, which is a biologically active material, is increased specifically in the fraction of extracellular vesicles released into the culture medium, and that the production amount of extracellular vesicles having a large size (having a particle diameter of about 800 nm or more and a volume of 10⁹ nm³ or more) was specifically increased by the treatment of inactivating Rab GTPase. The above-described phenomenon was also observed, when sonic hedgehog-expressing cells other than mesenchymal stem cells (for example, sonic hedgehog-expressing fibroblasts) were used. Furthermore, it was confirmed that extracellular vesicles whose production amount has been increased by the Rab18 inactivation treatment promotes angiogenesis, which is known to be the effect of sonic hedgehog.

From the aforementioned results, it is considered that when the treatment of inactivating Rab GTPase is performed on sonic hedgehog-expressing cells, the release of extracellular vesicles having a large particle diameter, which contain a large amount of sonic hedgehog, is promoted.

The present invention has been completed based on the aforementioned findings.

Specifically, the present invention includes the following (1) to (15).
(1) A method for producing extracellular vesicles, comprising obtaining extracellular vesicles secreted from cells subjected to a treatment of inactivating Rab, in particular, Rab18.
(2) The method according to the above (1), which is characterized in that the cells express sonic hedgehog.
(3) The method according to the above (1), which is characterized in that the cells are mesenchymal stem cells.
(4) The method according to the above (1), wherein the treatment of inactivating Rab GTPase is to treat with a Rab GTPase inhibitor.
(5) The method according to the above (1), wherein the treatment of inactivating Rab GTPase is to transfect of a Rab18-GDP mutant into the cells.
(6) The method according to any one of the above (1) to (5), which is characterized in that the extracellular vesicles contain sonic hedgehog.
(7) The method according to any one of the above (1) to (5), which is characterized in that the extracellular vesicle has a particle diameter of 800 nm or more and/or a volume of 10⁹ nm³ or more.
(8) A cell expressing sonic hedgehog, which is subjected to a treatment of inactivating Rab GTPase.
(9) The cell according to the above (8), which retains Hsp90 gene and nSMase2 gene in an expressible state.
(10) An extracellular vesicle containing sonic hedgehog and having a particle diameter of 800 nm or more and/or a volume of 10⁹ nm³ or more.
(11) A medicament or a pharmaceutical composition, comprising, as an active ingredient, the extracellular vesicle according to the above (10.
(12) A method for screening for a substance that inactivates Rab GTPase, comprising the following steps (a), and (b1), (b2) or (b3):
   (a) a step of allowing a candidate substance to come into contact with cells expressing sonic hedgehog, and
   (b1) a step of measuring the particle diameter and/or volume of extracellular vesicles secreted from the cells allowed to come into contact with the candidate substance in the step (a),
   (b2) a step of confirming the presence of absence of accumulation of sonic hedgehog or Hsp90 around the cell nuclei of the cells allowed to come into contact with the candidate substance in the step (a), or
   (b3) a step of confirming that the amount of KIF5A binding to Rab18 is decreased in the cells allowed to come into contact with the candidate substance in the step (a), or the amount of Hsp90 or nSMase is increased therein.
(13) The method according to the above (12), further comprising a step (c) of confirming whether the extracellular vesicles of the step (b1) contain sonic hedgehog.
(14) A method for screening a substance that activates Rab GTPase, comprising the following steps (d), and (e1) or (e2):
   (d) a step of allowing a candidate substance to come into contact with cells expressing sonic hedgehog, and
   (e1) a step of measuring the particle diameter and/or volume of extracellular vesicles secreted from the cells allowed to come into contact with the candidate substance in the step (d),
   (e2) a step of confirming that the expression level of sonic hedgehog or Hsp90 in the cytoplasm is increased and that the sonic hedgehog or the Hsp90 is diffusely present in the cytoplasm, or
   (e3) a step of confirming that the amount of KIF5A binding to Rab18 is increased in the cells allowed to come into contact with the candidate substance in the step (d), or the amount of Hsp90 or nSMase is increased therein.
(15) The method according to the above (14), further comprising a step (f) of confirming whether the extracellular vesicles of the step (e1) contain sonic hedgehog.

It is to be noted that the preposition "to" used in the present description indicates a numerical value range including the numerical values located left and right of the preposition.

### Advantageous Effects of Invention

According to the present invention, it becomes possible to efficiently produce extracellular vesicles containing a large amount of biologically active materials (for example, sonic hedgehog). As a result, it becomes easy to produce an extracellular vesicle drug that is useful as a therapeutic agent for various diseases.

### Brief Description of Drawings

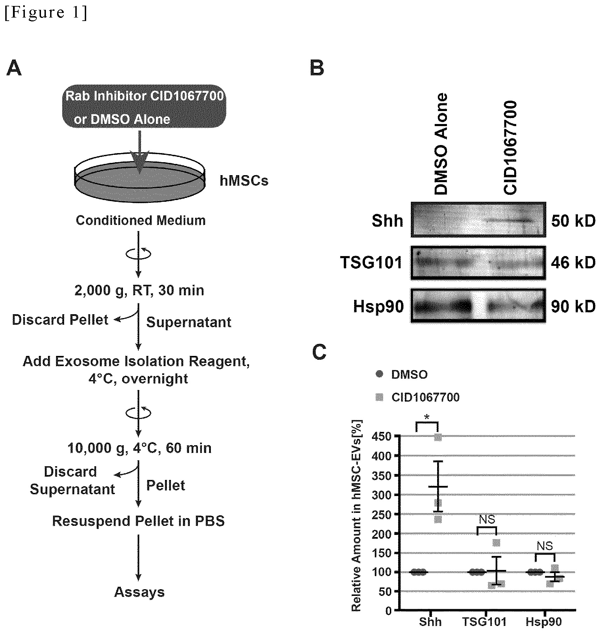

[Figure 1] Figure 1 shows the analysis of extracellular vesicles secreted from human mesenchymal stem cells treated with the Rab GTPase inhibitor, CID1067700. Figure 1A shows an overview of a method of isolating extracellular vesicles from the culture supernatant of human mesenchymal stem cells. Figure 1B shows the results obtained by performing immunoblotting on extracellular vesicles (hMSC-EVs) derived from human mesenchymal stem cells treated with CID1067700 or hMSC-EVs treated with DMSO (the solvent for CID1067700) alone, using an anti-Shh antibody, an anti-TSG101 antibody or an anti-Hsp90 antibody. Figure 1C shows the results obtained by quantifying the results of the immunoblotting of Figure 1B. NS, p ≥ 0.05; *p < 0.05; Welch's t test, n = 3. In all figures, data are shown as a mean value ± standard error.

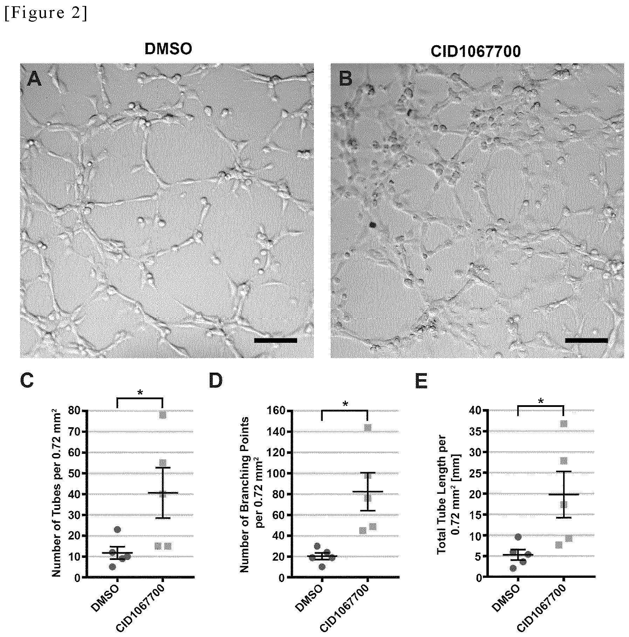

[Figure 2] Figure 2 shows promotion of HUVEC cell angiogenesis activity by extracellular vesicles secreted from human mesenchymal stem cells treated with CID1067700. Figures 2A and 2B show microscopic observation images obtained 90 minutes after the plating of HUVEC cells stimulated by hMSC-EVs produced from human mesenchymal stem cells treated with CID1067700 (B) or from human mesenchymal stem cells treated with DMSO alone (A). The scale bar indicates 100 µm. Figures 2C to 2E show the results obtained by quantifying the level of angiogenesis, using, as indicators, the number of formed tubes, the number of branch points, or the length of the tube. *p < 0.05, Welch's t test, n = 5.

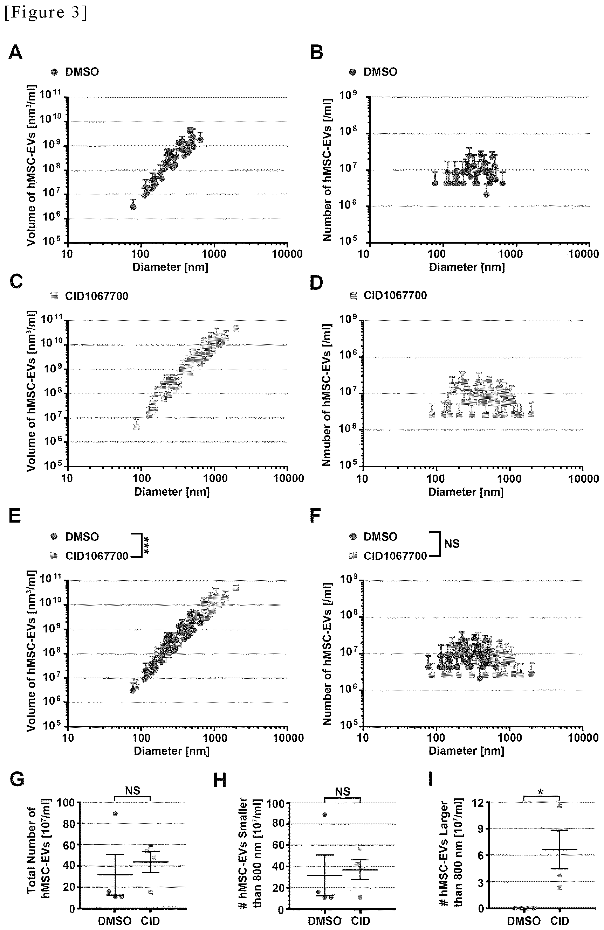

[Figure 3] Figure 3 shows the nanoparticle tracking analysis of hMSC-EVs secreted by a CID1067700 treatment. These are the results obtained by performing the nanoparticle tracking analysis on the volume of extracellular vesicles present in 1 mL of the culture supernatant of human mesenchymal stem cells (Figures 3A, 3C, and 3E), and the number of extracellular vesicles (Figures 3B, 3D, and 3F). The circle (**●**) in Figures 3A, 3B, 3E, and 3F indicates the results obtained using the culture supernatant of human mesenchymal stem cells treated with DMSO alone, whereas the square (■) in Figures 3C, 3D, 3E, and 3F indicates the results obtained using the culture supernatant of human mesenchymal stem cells treated with CID1067700. ***p < 0.001; Welch's t test. Data were obtained from three independent experiments. According to the CID1067700 treatment, the number of extracellular vesicles with a particle diameter of greater than 800 nm was significantly increased. These are the results obtained by statistically processing the results of the nanoparticle tracking analysis, and calculating the total number of extracellular vesicles (Figure 3G), the number of extracellular vesicles with a particle diameter of 100 to 800 nm (Figure 3H), and the number of extracellular vesicles with a particle diameter of >800 nm (Figure 3I). NS, p ≥ 0.05; *p < 0.05; Welch's t test, n = 4.

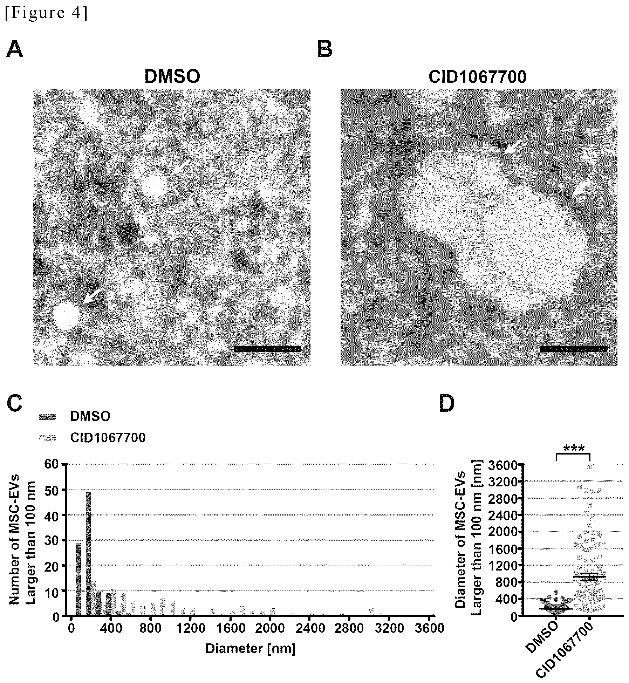

[Figure 4] Figure 4 shows the analysis of extracellular vesicles whose secretion is increased by a CID1067700 treatment. Figures 4A and 4B show transmission electron microscopic (TEM) observation images of hMSC-EV pellets obtained by a CID1067700 treatment (Figure 4B) or by a treatment with DMSO alone (Figure 4A). The scale bar indicates 1 µm. The arrow indicates extracellular vesicles with a low electron density. Figures 4C and 4D show the results of the statistical analysis of the diameters of hMSC-EVs observed by TEM. Figures 4C and 4D show a histogram (Figure 4C) and a scattered plot (Figure 4D) of the number of hMSC-EVs with diameter of larger than 100 nm. ***p < 0.001; Welch's t-test, n = 100.

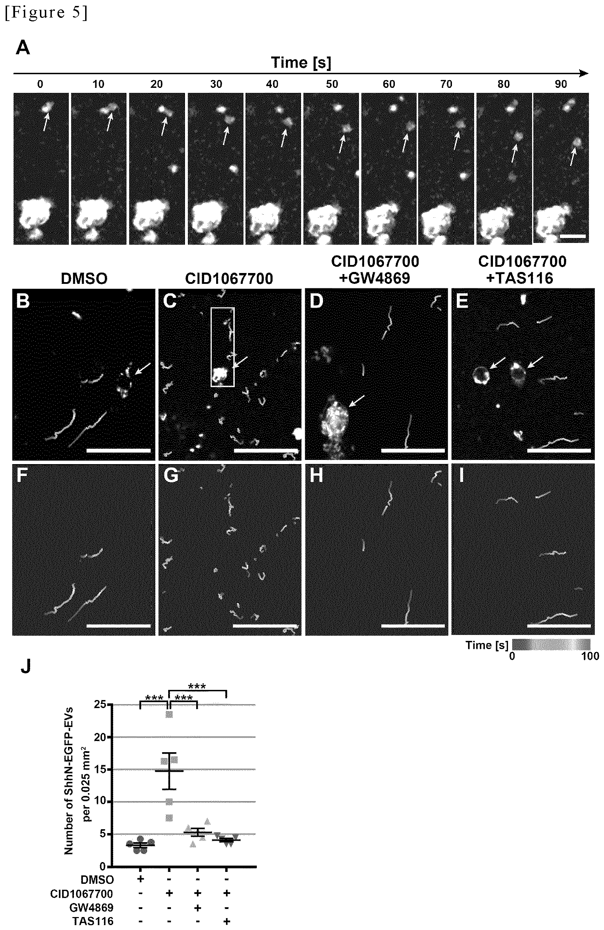

[Figure 5] Figure 5 shows the involvement of nSMase and Hsp90 in promotion of Shh-EV production by CID1067700. Figures 5A to 5I show the time-lapse fluorescence images of ShhN-EGFP-transduced NIH3T3 cells treated with CID1067700 (40 µM for 24 hours) and obtained by fluorescence microscopy (Figure 5A), and the results of nanoparticle tracking analysis in the absence (Figures 5B and 5F) or the presence (Figures 5C-5E and 5G-5I) of CID1067700. Figures 5D and 5H show the analysis results in the presence of an nSMase inhibitor GW4896 (1.25 µM, 24 hours), and Figures 5E and 5I show the analysis results in the presence of an Hsp90 inhibitor TAS116 (0.5 µM, 48 hours). Figures 5B to 5E show the results with the fluorescent images of cells, and Figures 5F to 5I show the results with no fluorescent images. The scale bar indicates 10 µm (Figure 5A) and 50 µm (Figures 5B-5I). The transduction efficiency of ShhN-EGFP was almost 100%. Figure 5J shows the results obtained by quantifying the number of Shh-EVs moving randomly in a 0.025 mm² field of view. ***p < 0.001; One-way ANOVA, n = 5.

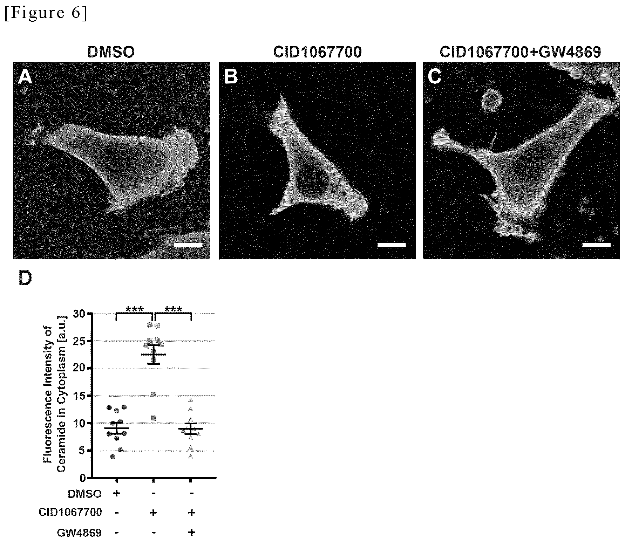

[Figure 6] Figure 6 shows studies regarding the influence of CID1067700 on the production amount of ceramide. In Figures 6A to 6C, cells were treated with DMSO alone (Figure 6A), CID1067700 (40 µM) (Figures 6B and 6C), and GW4869 (2.5 µM) for 24 hours. The scale bar indicates 10 µm. Figure 6D shows the results obtained by calculating the fluorescence signal intensity derived from ceramide present in cytoplasm according to statistical processing. ***p < 0.001; One-way ANOVA; n = 10. The increase in the production amount of ceramide by the CID1067700 treatment was offset by the nSMase inhibitor treatment.

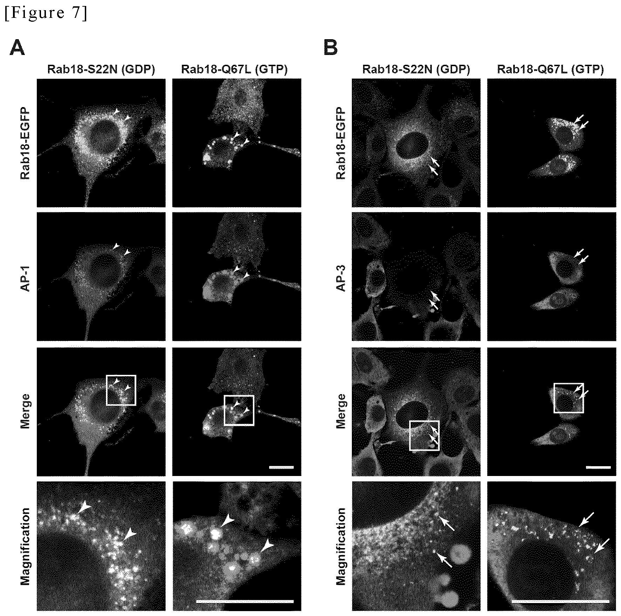

[Figure 7] Figure 7 shows observation of the intracellular localization of Rab18, AP-1 and AP-3. Figures 7A and 7B show fluorescence microscopic images of the immunostaining of AP-1 (Figures 7A) or AP-3 (Figures 7B) in NIH3T3 transfected with Rab18S22N-EGFP, which mimics the GDP-bound form, and with Rab 18Q67L-EGFP, which mimics the GTP-bound form. The arrowhead indicates co-localization of a Rab18 mutant and AP-1. The arrow indicates perinuclear accumulation of AP-3 in Rab18S22N-EGFP overexpressing cells. The scale bar indicates 20 µm.

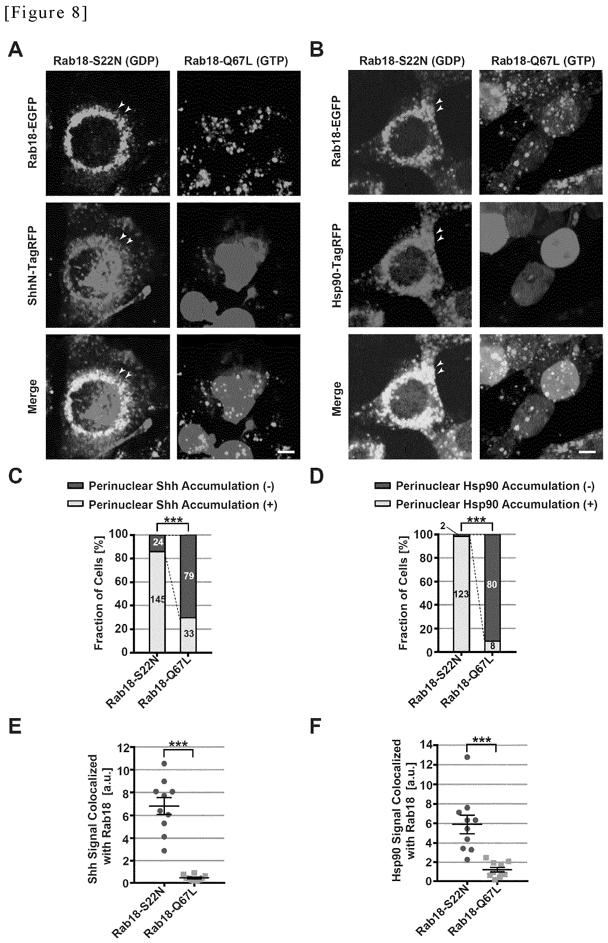

[Figure 8] Figure 8 shows the relationship between the intracellular distribution of Shh and Hsp90, and the nucleotide state of Rab18. Figures 8A and 8B show fluorescence images of NIH3T3 cells doubly transduced with nucleotide-state mutants, EGFP-Rab18S22N (GDP-bound form; green) or EGFP-Rab18Q67L (GTP-bound form; green), Shh-tagRFP (red) or Hsp90-tagRFP (red). The scale bar indicates 10 µm. The arrow indicates Rab18-GDP and Shh-tagRFP (Figure 8A) or Rab18-GDP and Hsp90-tagRFP (Figure 8B) that are co-localized around the cell nucleus. Figures 8C and 8D show the results obtained by calculating according to statistical processing the percentage (%) of cells in which Rab18 mutant and Shh-tagRFP are co-localized or are not co-localized (Figure 8C), and the percentage (%) of cells in which Rab18 mutant and Hsp90-tagRFP are co-localized or are not co-localized (Figure 8D). ***p < 0.001; chi-square test; number of cells, n = 88-169. Figures 8E and 8F show the results obtained by calculating according to statistical processing the mean fluorescence intensities of Shh-tagRFP (Figure 8E) and Hsp90-tagRFP (Figure 8F) for Rab18 positive pixels. Rab18-GDP significantly recruits Shh and Hsp90. ***p < 0.001; Welch's t-test; n = 10.

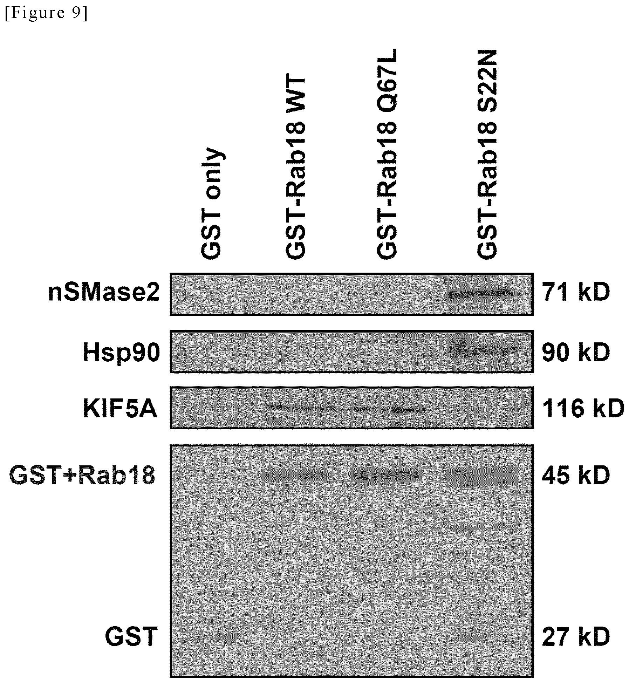

[Figure 9] Figure 9 shows the results of mouse brain lysates pulled down by GST-Rab18WT, GST-Rab18 Q67L, and GST-Rab18 S22N. These are the results of immunoblotting performed on sediments pulled down from mouse brain lysates by GST-tagged Rab18 nucleotide mutant. Similar results were obtained in three experiments. Rab18S22N (GDP) specifically interacts with nSMase2 and Hsp90, while wild-type Rab18 and Rab18Q67L (GTP) specifically interact with KIF5A kinesin.

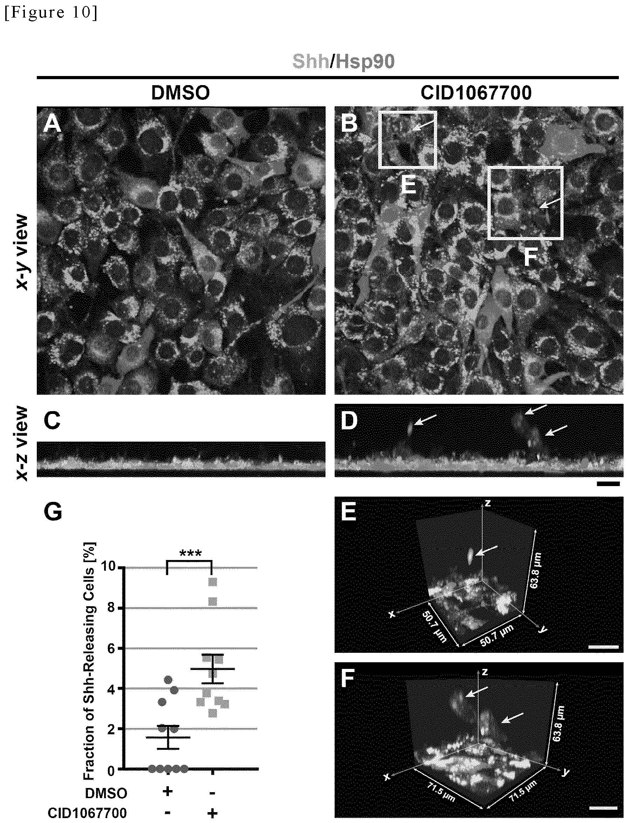

[Figure 10] Figure 10 shows the analysis of structures released from cells by a CID1067700 treatment. Figure 10A to 10F shows fluorescence images of live NIH3T3 cells expressing ShhN-EGFP (green) and Hsp90-TagRFP (red), which are treated with DMSO (Figures 10A and 10B) or with CID1067700 (40 µM) (Figures 10B and 10D-10F) for 24 hours and are then observed by fluorescence microscopy. Figures 10A and 10B are x-y images, Figures10C and 10D are x-z images, and Figures 10E and 10F are three-dimensionally reconstructed images. The scale bar indicates 20 µm. The arrow indicates an extracellular region comprising Shh and Hsp90. Figure 10G shows the results obtained by calculating according to statistical processing the percentage of cells releasing Shh/Hsp90 in cells treated with CID1067700 or with DMSO alone. ***p < 0.001; Welch's t-test.

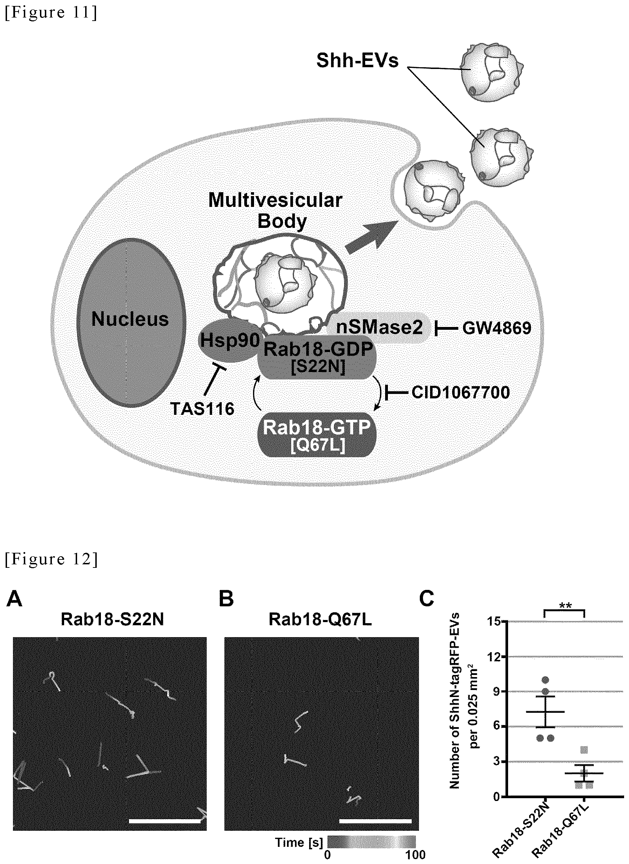

[Figure 11] Figure 11 shows a conceptual view of the process of releasing Shh-EVs by inactivation of Rab18GTPase. This is a conceptual view showing a Rab18 GTPase cycle that regulates Shh-EV production by specifically incorporating Shh, nSMase2 and Hsp90 into the perinuclear multivesicular bodies to produce extracellular vesicles. TAS116, GW4869, and CID1067700 inhibit Hsp90, nSMase2, and Rab18, respectively. The Rab18S22N and Rab18Q67L mutants are mutants that mimic the activity of GDP-bound Rab18 GTPase and GTP-bound Rab18 GTPase, respectively.
[Figure 12] Figure 12 shows the relationship between the GTPase cycle of Rab18 and Shh-EV production. Figures 12A and 12B show the nanoparticle tracking of ShhN-tagRFP-transduced NIH3T3 cells, which were transfected with EGFP-Rab18S22N (Figure 12A) and EGFP-Rab18Q67L (Figure 12B). The scale bar indicates 50 µm. Figures 12C shows the results obtained by quantifying the number of Shh-EVs moving randomly in a 0.025 mm² field of view. ***p < 0.001; One-way ANOVA; n = 5. **p < 0.01; Welch's t test; n = 4.

### Description of Embodiments

Hereafter, the embodiments for carrying out the present invention will be described. It is to be noted that when "the present embodiment" is mentioned, it refers to all embodiments described in the present description, unless otherwise indicated.

A first embodiment relates to a method for producing extracellular vesicles, comprising obtaining extracellular vesicles secreted from cells subjected to a treatment of inactivating Rab GTPase, in particular, Rab18.

In the present embodiment, inactivation of Rab GTPase will be explained using Rab18 as an example. The treatment of inactivating Rab18 means a treatment of suppressing or inhibiting the activity of a GTP-bound form of Rab 18 (Rab18-GTP). Specifically, it is a treatment of converting Rab18 in cells to GDP-bound Rab18, and for example, this treatment can be achieved by treating cells with a GTPase inhibitor such as CID1067700. Alternatively, inactivation of Rab18 in cells can also be achieved by overexpression of a mutant mimicking GDP-bound Rab18 (e.g., Rab18 S22N (Rab18 mutant in which the serine at the 22nd amino acid residue is substituted with asparagine), etc.).

In the present embodiment, the cells that are preferably used to produce extracellular vesicles are cells expressing sonic hedgehog (Shh; hereinafter also referred to as "Shh"). The cells expressing Shh may include, for example, mesenchymal stem cells. Mesenchymal stem cells (MSCs) are a type of somatic stem cells having an ability to differentiate into mesoderm-derived tissues (e.g., bone, cartilage, fats, blood vessels, cardiomyocytes, etc.), neurons and glia derived from the ectoderm, and stem cells derived from the endoderm, etc. Human mesenchymal stem cells are characterized in that, for example, they are positive for cell surface markers such as CD73 and CD105, and are negative for myeloid markers such as CD34, CD14, CD45 and MHC class II antigen. Mesenchymal stem cells can be collected from bone marrow, dental pulp or fats, or commercially available mesenchymal stem cells can also be obtained.

Furthermore, the cells expressing Shh may be any established cells other than mesenchymal stem cells, into which an Shh-expressing vector has been introduced and which are in a state in which Shh can be expressed. Examples of such cells expressing Shh may include the Shh-expressing NIH3T3 cells described in the after-mentioned example. Further, the cells expressing Shh may be cells into which each expression vector expressing the hsp90 gene and/or the nSMase2 gene, as well as the Shh gene are introduced and which are in a state in which the expression of these genes can be increased.

The inventors have confirmed that, in the fraction of extracellular vesicles secreted from cells subjected to a treatment of inactivating Rab GTPase, in particular, Rab18, the abundance ratio of Shh-containing extracellular vesicles is high. In addition, when the Shh-expressing cells are subjected to a treatment of inactivating Rab18, extracellular vesicles having a particle diameter of 800 nm or more and/or a volume of 10⁹ nm³ or more are secreted. Based on these findings, it is considered that when cells are subjected to the treatment of inactivating Rab18, secretion of extracellular vesicles containing a large amount of Shh and having a particle diameter of 800 nm or more and/or a volume of 10⁹ nm³ or more is promoted. It is to be noted that the particle diameter and volume of extracellular vesicles can be measured by methods such as a nanoparticle tracking analysis method (NTA, Filipe et al. Pharmaceutical Research, 27, 796-810, 2010), a light scattering method (e.g., a dynamic light scattering (DLS) method), and an electron microscopic observation method. Herein, the "particle diameter of 800 nm or more" and the "volume of 10⁹ nm³ or more" are calculated by nanoparticle tracking analysis. Therefore, the values measured by other methods are desirably converted based on the values measured by the nanoparticle tracking analysis method.

Extracellular vesicles secreted from cells can be isolated by methods publicly known in the present technical field. For example, the medium, in which the cells subjected to the Rab GTPase inactivation treatment have been cultured, is collected, and a culture supernatant obtained by removing the cells and the residues from the collected medium is concentrated by ultrafiltration, etc., as necessary, followed by performing, for example, an ultracentrifugation method, a density gradient centrifugation method, size exclusion chromatography, etc., singly or in combination, so that desired extracellular vesicles can be isolated and purified. Moreover, extracellular vesicles having a particle diameter of 800 nm or more and/or a volume of 10⁹ nm³ or more, which contain a large amount of Shh, can be isolated by methods publicly known in the present technical field. For example, the extracellular vesicle fraction can be separated by a fractional centrifugation method, size exclusion chromatography, a filtration method using a filtration membrane with a suitable pore size, a density gradient centrifugation method, flow cytometry, etc. Furthermore, since Shh has been reported to also exist on the surface of extracellular vesicles (Tanaka et al., Nature 435, 172-177, 2005), an immunoaffinity separation method using an antibody against Shh, or flow cytometry, is combined with the aforementioned methods, and the extracellular vesicles having a particle diameter of 800 nm or more and/or a volume of 10⁹ nm³ or more, which contain a large amount of Shh, can be separated by the thus combined method.

A second embodiment relates to a cell expressing sonic hedgehog, which is subjected to a treatment of inactivating Rab GTPase, in particular, Rab 18.

Regarding the "cell expressing sonic hedgehog" and the "treatment of inactivating Rab GTPase" in the second embodiment, please refer to the explanation for the first embodiment.

A third embodiment relates to an extracellular vesicle containing sonic hedgehog and having a particle diameter of 800 nm or more and/or a volume of 10⁹ nm³ or more. The extracellular vesicle according to the third embodiment can be produced, for example, by the method according to the first embodiment. To date, there have been no reports regarding such an extracellular vesicle containing sonic hedgehog and having a particle diameter of 800 nm or more and/or a volume of 10⁹ nm³ or more. In addition, the extracellular vesicle produced by the method according to the first embodiment has physiological activity such as promotion of angiogenesis. Accordingly, the extracellular vesicle according to the third embodiment can be utilized as a medicament or a pharmaceutical composition.

Thus, a fourth embodiment relates to a medicament or a pharmaceutical composition, comprising, as an active ingredient, the extracellular vesicle containing sonic hedgehog and having a particle diameter of 800 nm or more and/or a volume of 10⁹ nm³ or more (i.e., the extracellular vesicle according to the third embodiment). As mentioned above, since the extracellular vesicle according to the third embodiment promotes angiogenesis, this extracellular vesicle is considered to exhibit effects on the treatment of ischemia (ischemic heart disease, cerebral ischemia, etc.), on the treatment in regenerative medicine after ischemia, on the treatment of peripheral circulatory disorders, etc. Moreover, it has been reported that the extracellular vesicle containing Shh is involved in spinal cord injury healing (Jia et al., Regen Ther. 8:309-315. doi: 10.1016/j.reth.2021.08.007. eCollection 2021; Jia et al., Acta Neurochir (Wien) . 163: 2297-2306 doi: 10.1007/s00701-021-04829-9. Epub 2021 Apr 5.) or wound healing (Roefs et al., Trends in Cell Biology 30, No. 12. 9901013-https://doi.org/10.1016/j.tcb.2020.09.009). Furthermore, it has also reported that Lamellipodia do not move well in schizophrenia models, but that the extracellular vesicle containing Shh activates the movement of the Lamellipodia (Nishimura et al., Developmental Cell 56, 842-859, March 22, 2021). Accordingly, the extracellular vesicle according to the third embodiment is considered to also exhibit effects on the treatment of wounds and schizophrenia.

The extracellular vesicle according to the third embodiment itself may be administered. In general, however, the present extracellular vesicle may be administered in the form of a pharmaceutical composition comprising one or two or more pharmaceutical additives, as well as the present extracellular vesicle as an active ingredient. In addition, the medicament or the pharmaceutical composition according to the present embodiment may also comprise other known ingredients effective for the treatment of diseases to be treated, as well as the extracellular vesicle according to the third embodiment.

Examples of the dosage form of the medicament or pharmaceutical composition according to the present embodiment may include a tablet, a capsule, a granule, a powder agent, a syrup agent, a suspending agent, a suppository, an ointment, a cream agent, a gelling agent, a patch, an inhalant, an injection, and nasal drops. These formulations are prepared according to ordinary methods. In the case of a liquid formulation, it may be dissolved or suspended in water or other suitable solvents upon use. In addition, a tablet or a granule may be coated according to publicly known methods. In the case of an injection, it is prepared by dissolving the active ingredient and the like in water, etc. Such an injection may also be prepared by dissolving the present active ingredient and the like in a normal saline or a glucose solution, as necessary. Otherwise, a buffer or a preservative may be added to the injection.

A formulation for use in oral administration or parenteral administration is provided in the form of any given formulation. Examples of the formulation form may include: medicaments or pharmaceutical compositions for oral administration, having forms such as a granule, a fine granule, a powder agent, a hard capsule, a soft capsule, a syrup agent, an emulsion, a suspending agent, or a liquid agent; injections for intravenous, intramuscular or subcutaneous administration, etc.; and medicaments or pharmaceutical compositions for parenteral administration, having forms such as an infusion, a transdermal absorbent, a transmucosal absorbent, nasal drops (nasal spray, etc.), an inhalant, or a suppository. An injection, an infusion, or the like can also be used by being prepared in the dosage form of powders, such as a freeze-dried form, and then dissolving the powders in a suitable aqueous medium such as a normal saline, upon use.

The types of pharmaceutical additives used in production of the medicament or pharmaceutical composition according to the present embodiment, the percentage of the pharmaceutical additives to the active ingredient, or the method for producing the medicament or pharmaceutical composition can be selected, as appropriate, by a person skilled in the art, depending on the form. As pharmaceutical additives, inorganic or organic substances, or solid or liquid substances can be used. In general, such pharmaceutical additives can be mixed into the present medicament or pharmaceutical composition, in an amount between 1% by weight and 90% by weight, with respect to the weight of the active ingredient. Specific examples of the pharmaceutical additives may include lactose, glucose, mannit, dextrin, cyclodextrin, starch, sucrose, magnesium aluminometasilicate, synthetic aluminum silicate, carboxymethyl cellulose sodium, hydroxypropyl starch, carboxymethyl cellulose calcium, ion exchange resin, methyl cellulose, gelatin, gum Arabic, hydroxypropyl cellulose, hydroxypropylmethyl cellulose, polyvinyl pyrrolidone, polyvinyl alcohol, light anhydrous silicic acid, magnesium stearate, talc, tragacanth, bentonite, beegum, titanium oxide, sorbitan fatty acid ester, sodium lauryl sulfate, glycerin, fatty acid glycerin ester, purified lanolin, glycerogelatin, polysorbate, macrogol, vegetable oil, wax, liquid paraffin, white petrolatum, fluorocarbon, nonionic surfactant, propylene glycol, and water.

In order to produce a solid formulation for oral administration, the active ingredient is mixed with excipient components such as, for example, lactose, starch, crystalline cellulose, calcium lactate, or anhydrous silicic acid to prepare a powder agent. Otherwise, as necessary, binders such as white sugar, hydroxypropyl cellulose or polyvinyl pyrrolidone, disintegrators such as carboxymethyl cellulose or carboxymethyl cellulose calcium, and the like are further added to the aforementioned mixture, and the thus obtained mixture is then subjected to wet or dry granulation, so as to prepare a granule. In order to produce a tablet, these powder agents and granules may be directly subjected to tablet making, or these powder agents and granules, together with a lubricant such as magnesium stearate or talc, may be subjected to tablet making. These granules or tablets can be processed into enteric-coated formulations by being coated with enteric-coating base agents such as hydroxypropylmethyl cellulose phthalate or a methacrylic acid-methyl methacrylate polymer, or can also be processed into sustained release formulations by being coated with ethyl cellulose, carnauba wax, hydrogenated oil, or the like. In addition, in order to produce a capsule agent, a powder agent or a granule is filled into a hard capsule, or the active ingredient is directly coated with gelatin, or is first dissolved in glycerin, polyethylene glycol, sesame oil, olive oil or the like, and is then coated with a gelatin film, so as to prepare a soft capsule.

In order to produce an injection, the active ingredient is dissolved in distilled water for injection, as necessary, together with a pH adjuster such as hydrochloric acid, sodium hydroxide, lactose, lactic acid, sodium, sodium monohydrogen phosphate or sodium dihydrogen phosphate, and a tonicity agent such as sodium chloride or glucose, and thereafter, the obtained solution is subjected to aseptic filtration, and is then filled into an ampule. Otherwise, mannitol, dextrin, cyclodextrin, gelatin or the like is further added to the resulting solution, followed by vacuum freeze drying, so as to prepare an injection that is soluble at the time of use. Alternatively, lecithin, polysorbate 80, polyoxyethylene hydrogenated castor oil, or the like is added to the active ingredient for emulsification in water, so as to prepare an emulsion for injection.

In order to produce a rectal administration agent, the active ingredient, together with a suppository base agent such as cacao butter, fatty acid tri-, di- and mono-glyceride, or polyethylene glycol, is humidified and is dissolved, and the resultant is then poured into a mold, followed by cooling. Otherwise, the active ingredient may be dissolved in polyethylene glycol, soybean oil, or the like, and may be then coated with a gelatin film.

The applied dose and the number of doses of the medicament or pharmaceutical composition according to the present embodiment are not particularly limited. The applied dose and the number of doses can be appropriately selected according to the judgment of a doctor or a pharmacist, depending on conditions such as the purpose of preventing and/or treating deterioration and/or progression of a treatment target disease, the type of the disease, and the body weight and age of a patient.

Generally, the dose applied for an adult per day by oral administration is approximately 0.01 to 1000 mg (the weight of the active ingredient), and this dose can be administered once or divided over several administrations per day, or every several days. In the case of using the medicament or the pharmaceutical composition as an injection, the injection is desirably administered to an adult continuously or intermittently at a daily dose of 0.001 to 100 mg (the weight of the active ingredient).

The medicament or pharmaceutical composition according to the present embodiment can be prepared as a sustained release formulation, such as an implant tablet and a delivery system encapsulated into a microcapsule, by using a carrier capable of preventing the prompt removal of the agent from the body. As such carriers, biodegradable and biocompatible polymers, such as ethylene vinyl acetate, polyanhydride, polyglycolic acid, collagen, polyorthoester and polylactic acid, can be used. These materials can be easily prepared by a person skilled in the art. Moreover, a liposome suspension can also be used as a pharmaceutically acceptable carrier. Liposome is prepared as a lipid composition comprising, but are not limited thereto, phosphatidylcholine, cholesterol and PEG-derived phosphatidylethanol (PEG-PE), by being passed through a filter with a suitable pore size, so that it can have a size suitable for the use thereof, and it is then purified by a reverse phase evaporation method.

The medicament or pharmaceutical composition according to the present embodiment may be provided in the form of a kit, together with an instruction manual regarding an administration method and the like. The drug included in the kit is supplied with a vessel, in which the activity of the components of the medicament or the pharmaceutical composition is effectively sustained for a long period of time, the drug and the like are not adsorbed on the inside thereof, and the vessel is produced from materials that do not degrade the components. For example, a sealed glass ampule may comprise a buffer or the like that has been enclosed in the presence of neutral and unreactive gas such as nitrogen gas.

Moreover, an instruction manual may be included with the kit. The instruction manual of the kit may be printed on a paper or the like, or may also be stored in an electromagnetically readable medium such as CD-ROM or DVD-ROM, and may be then supplied to users.

A fifth embodiment relates to a method comprising administering the medicament or pharmaceutical composition according to the fourth embodiment to a therapeutic subject, so as to treat or prevent ischemia (ischemic heart disease, cerebral ischemia, etc.), regenerative medicine after ischemia, peripheral circulatory disorders, wounds, neuropsychiatric diseases such as schizophrenia, renal failure, diabetes, steatohepatitis, etc.

The "mammal" as a therapeutic or preventive subject means any given animal classified into mammals, and thus, is not particularly limited. Examples of the mammal used herein may include: humans; pet animals such as dogs, cats and rabbits; and livestock animals such as bovines, pigs, sheep and horses. A particularly preferable "mammal" is a human.

A sixth embodiment relates to a method for screening for a substance that inactivates Rab GTPase, in particular, Rab18, the method comprising the following steps (a), and (b1), (b2) or (b3). In some cases, the present screening method may further comprise the following step (c):
(a) a step of allowing a candidate substance to come into contact with cells expressing sonic hedgehog, and
(b1) a step of measuring the particle diameter and/or volume of extracellular vesicles secreted from the cells allowed to come into contact with the candidate substance in the step (a),
(b2) a step of confirming the presence of absence of accumulation of sonic hedgehog or Hsp90 around the cell nuclei of the cells allowed to come into contact with the candidate substance in the step (a), or
(b3) a step of confirming that the amount of KIF5A binding to Rab18 is decreased in the cells allowed to come into contact with the candidate substance in the step (a), or the amount of Hsp90 or nSMase is increased therein, and optionally,
(c) a step of confirming whether the extracellular vesicles of the step (b1) (i.e., the extracellular vesicles whose particle diameter and/or volume have been measured in the step (b1)) contain sonic hedgehog.

The substance that inactivates Rab GTPase means a substance that suppresses or inhibits the activity of a GTP-bound form (Rab-GTP) of Rab GTPase, and it is, for example, a substance that converts Rab GTPase to a GDP-bound form, such as the inhibitor CID1067700. When CID1067700 is, for example, added to the medium and it is thereby allowed to come into contact with the cells expressing Shh, the extracellular vesicles having a particle diameter of 800 nm or more and/or a volume of 10⁹ nm³ or more, which contain Shh, appear in the extracellular vesicle fraction secreted from the cells (see the Examples later). Accordingly, when a candidate substance is allowed to come into contact with the cells expressing Shh, and the amount of the extracellular vesicles having a particle diameter of 800 nm or more and/or a volume of 10⁹ nm³ or more and containing Shh secreted from the cells is thereby increased, it can be determined that the candidate substance is likely to be a Rab GTPase-inactivating substance. In addition, when a candidate substance is allowed to come into contact with the cells expressing Shh, and Shh or Hsp90 is thereby accumulated around the cell nuclei, it can be determined that the candidate substance is likely to be a Rab GTPase-inactivating substance.

Moreover, when the Rab GTPase is inactivated, the binding amount of KIF5A, the GTP effector to Rab, is decreased and the binding amount of Hsp90 or nSMase, the GDP effector, is increased. Therefore, when the binding amount of KIF5A to Rab18 is decreased or the binding amount of Hsp90 or nSMase is increased after the aforementioned step (a), it can be determined that the candidate substance is a Rab18-inactivating substance. The Rab18-bound effector can be identified, for example, by immunoblotting (using KIF5A, or an antibody against Hsp90 or nSMase) against immunoprecipitates of Rab18, or against pulldown assay precipitates obtained using GST-Rab18. Identification of binding specific to GTP-bound Rab18 or GDP-bound Rab18 can be easily accomplished by using a Rab18Q67L or Rab18S22N point mutant, or by mixing reagents such as GTPγS or GDPβS into the reaction solution (For details, see the Examples later).

A seventh embodiment relates to use of a substance that inactivates Rab GTPase. When Rab GTPase, in particular, Rab18 is inactivated in a living body, secretion of extracellular vesicles containing a large amount of Shh is promoted. Thus, the Rab GTPase-inactivating substance (for example, a Rab18-inactivating substance) can be used to prevent or treat ischemia (ischemic heart disease, cerebral ischemia, etc.), regenerative medicine after ischemia, peripheral circulatory disorders, wounds, schizophrenia, etc.

An eighth embodiment relates to a method for screening a substance that activates Rab GTPase, in particular, Rab18, the method comprising the following steps (d), and (e1) or (e2). In some cases, the present screening method may further comprise the following step (f):
(d) a step of allowing a candidate substance to come into contact with cells expressing sonic hedgehog, and
(e1) a step of measuring the particle diameter and/or volume of extracellular vesicles secreted from the cells allowed to come into contact with the candidate substance in the step (d),
(e2) a step of confirming that the expression level of sonic hedgehog or Hsp90 in the cytoplasm is increased and that the sonic hedgehog or the Hsp90 is diffusely present in the cytoplasm, or
(e3) a step of confirming that the amount of KIF5A binding to Rab18 is increased in the cells allowed to come into contact with the candidate substance in the step (d), or the amount of Hsp90 or nSMase is increased therein, and optionally,
(f) a step of confirming whether the extracellular vesicles of the step (e1) (i.e., the extracellular vesicles whose particle diameter and/or volume have been measured in the step (e1)) contain sonic hedgehog.

The substance that activates Rab GTPase means a substance that activates a GDP-bound form (Rab-GDP) of Rab GTPase, and it is, for example, a substance that converts Rab GTPase to a GTP-bound form, such as a GDP-GTP substitution promoting agent. When a GTP mutant of Rab18, Rab18-Q67L, is introduced into the cells expressing Shh, the number of the extracellular vesicles having a particle diameter of 800 nm or more and/or a volume of 10⁹ nm³ or more and containing Shh, contained in the extracellular vesicle fraction secreted from the cells, is decreased (see the Examples later). Accordingly, when a candidate substance is allowed to come into contact with the cells expressing Shh, and the amount of the extracellular vesicles having a particle diameter of 800 nm or more and/or a volume of 10⁹ nm³ or more and containing Shh secreted from the cells is thereby decreased, it can be determined that the candidate substance is likely to be a Rab18-activating substance. In addition, when a candidate substance is allowed to come into contact with the cells expressing Shh, and thereby, the expression level Shh or Hsp90 is increased and the Shh or the Hsp90 is diffusely present in the cytoplasm, it can be determined that the candidate substance is likely to be a Rab18-activating substance.

Moreover, when the Rab GTPase is activated, the binding amount of KIF5A, the GTP effector to Rab, is increased and the binding amount of Hsp90 or nSMase, the GDP effector, is decreased. Therefore, when the binding amount of KIF5A to Rab18 is increased or the binding amount of Hsp90 or nSMase is decreased after the aforementioned step (d), it can be determined that the candidate substance is a Rab18-activating substance. The Rab18-bound effector can be identified, for example, by immunoblotting (using KIF5A, or an antibody against Hsp90 or nSMase) against immunoprecipitates of Rab18, or against pulldown assay precipitates obtained using GST-Rab18 (For details, see the Examples later).

A ninth embodiment relates to use of a substance that activates Rab GTPase. When a substance that activates Rab GTPase, in particular, Rab18, is allowed to come into contact with the cells expressing Shh, section of extracellular vesicles containing Shh from the cells is suppressed or inhibited. It has been reported that Shh contained in extracellular vesicles secreted from cancer-related fibroblasts promotes proliferation and movement (metastasis) of cancer cells (Zhao et al., Cancer Medicine. 9: 2500-2513, 2020). Therefore, it is considered that proliferation of cancers (e.g., esophageal cancer, etc.) and/or metastasis of cancer cells can be suppressed and cancer can be prevented or treated by allowing a Rab18-activating substance to come into contact with cells secreting Shh, which are present in a cancer environment in a living body.

As described above, a Rab GTPase-activating substance (e.g., a Rab18-activating substance) can be used as a means for a method for suppressing the release of extracellular vesicle containing Shh, and can also be used for the prevention or treatment of cancer.

When the present description as a whole includes singular terms with the articles "a," "an," and "the," these terms include not only single items but also multiple items, unless otherwise clearly specified from the context that it is not the case. In addition, in the present description, the term "about" or "approximately" is used to mean a numerical range of ±10%.

Hereinafter, the present invention will be further described in the following examples. However, these examples are only illustrative examples of the embodiments of the present invention, and thus, are not intended to limit the scope of the present invention.

### Examples

### 1. Experimental methods

### 1-1. Cells

Human mesenchymal stem cells (hMSCs) were purchased from Lonza (Basel, Switzerland, #PT-2501), and were cultured and characterized according to the protocols provided by the manufacturer. The human mesenchymal stem cells were cultured in Mesenchymal Stem Cells Basal Medium (Lonza, #PT3238) supplemented with supplements and growth factors in a 10-cm dish (Thermo Scientific, #150466). A trypsin/EDTA solution (Lonza, #CC-3232) was added to the culture dish, followed by performing incubation at 37°C for 5 minutes, so that the cells were detached from the dish. After recovering the detached cells, they were seeded at a density of 5,000 to 6,000 cells/cm², and the cells were passaged every 7 days. It is to be noted that cells that had been passaged at a maximum of 6 times were used in the experiments. The medium was changed with a fresh one every three days.

HUVEC cells were purchased from Lonza (#C-2519AS), and were cultured in EGM-2 media (Lonza, #CC-5022) supplemented with supplements and growth factors. The cells were rinsed with a HEPES buffer (Lonza, #CC-5022), and thereafter, a trypsin/EDTA solution (Lonza, #CC-5012) was added to the resulting cells, followed by performing incubation at 37°C for 3-5 minutes, so that the cells were detached from the dish. The detached cells were recovered in a trypsin neutralization solution (Lonza, #CC-5002), were seeded at a density of approximately 2,500 cells/cm², and were passaged every 7 days. It is to be noted that cells that had been passaged at a maximum of 4 times were used in the experiments. The medium was replaced with a fresh one every 3 days. The HUVEC cells were plated in an 8-well LabTek II Chamber #1.5 German Cover Glass System (Thermo Scientific Nunc, Denmark, #155409) that had been pre-coated with Matrigel (Corning, #356231) for each passage, and were then used for the angiogenesis assay.

NIH3T3 cells were cultured in DMEM (High Glucose; FUJIFILM Wako Pure Chemical Co., Japan, #044-29765) containing 10% FBS (JRH Biosciences, #F7524, Lot #BCBT3482) according to a standard method. The NIH3T3 cells were transformed with a Rab-GFP plasmid, using Lipofectamine LTX-Plus reagent (Thermo Fisher), and were selected with 200 µg/ml G418 (Thermo Fisher, #10131035) and were then subcloned.

### 1-2. Antibodies

As anti-KIF5A antibody (#kif5a(n), RRID:AB_2571744, 1:1,000), a previously reported antibody was used (Kanai et al., The Journal of neuroscience 20, 6374-6384 200). Rabbit anti-Shh antibody (#20697-1-AP, RRID:AB_10694828, 1:1,000) was purchased from Proteintech. Rat anti-Hsp90 monoclonal antibody 16F1 (#ADI-SPA-835, RRID:AB_311881, 1:500) was purchased from Enzo Life Sciences. mouse anti-TSG101 monoclonal antibody 4A10 (# MA1-23296, RRID:AB_2208088, 1:1,000) was purchased from Thermo Fisher Scientific. Rabbit anti-GST antibody (#A5838, RRID:AB_258261, 1:1,000), mouse anti-γ-adaptin (AP-1) monoclonal antibody (#A4200, RRID:AB_476720, 1:1,000), mouse anti-AP-3 monoclonal antibody (#A4825, RRID:AB_258203, 1:1,000) and anti-ceramide antibody (#C8104, RRID:AB_259087, 1:500) were purchased from Sigma-Aldrich. Mouse anti-nSMase2 antibody (# sc-166637, RRID:AB_2270817, 1:1,000) was purchased from Santa Cruz Biotechnology.

For use as secondary antibodies, horseradish-peroxidase (HRP)-conjugated anti-rabbit IgG antibody and HRP-conjugated anti-mouse IgG antibody (1:1,000) were purchased from GE Healthcare Life Sciences, and Alexa-Fluor 405-, 488-, and 568-conjugated anti-mouse IgG, anti-rat IgG, anti-rabbit IgG, and anti-mouse IgM antibodies (1:200 to 1:500) were purchased from Thermo Fisher.

### 1-3. Expression vectors

In order to construct a Shh-N-EGFP expression vector and a Shh-N-TagRFP expression vector, 594 nucleotides (corresponding amino acids 1 to 198) of the N-terminus of mouse Shh cDNA (kindly provided by Dr. Andrew P. McMahon) were amplified by PCR and were then inserted into the HindIII-NcoI site of a pEGFP vector (#6077-1, Clontech). Thereafter, the Shh-N-inserted pEGFP vector was cleaved with HindIII and EcoRI to obtain a Shh-N-EGFP fragment. The ShhN-EGFP fragment was inserted into a pBK-CMV plasmid (#212209, Agilent Technologies) to construct a pShh-N-EGFP expression plasmid. Subsequently, the Shh-N cDNA was amplified by PCR using the following primers:
Sal-ShhATGKozak-fwd:
   5'-ACCGTCGACCATGGTGCTGCTGCTGGCCAGATG-3' (SEQ ID No: 1), and
Bam-Shh198-rev:
   5'-CAAGGATCCCGGCCGCCGGATTTGGCCGCCACG-3' (SEQ ID No: 2).

The amplified fragment was inserted into a pTagRFP-N vector (#FP142, Evrogen) to construct a Shh-N-tagRFP expression plasmid vector. These expression units were introduced into cells, using the ViraPower Adenoviral Expression System (Thermo Fisher) in accordance with the manufacturer's instruction manuals.

In order to construct an Hsp90 expression vector, mouse Hsp90aa1 cDNA was amplified from FANTOM clone #I1C0020P08 by PCR using the following primers:
Xho+hsp90aa1atgFw:
   5'-ACCCTCGAGCTATGCCTGAGGAAACCCAGACCCAAG3' (SEQ ID No: 3), and
Kpn+hsp90 aa1stopRev:
   5'-ACCGGTACCTTAGTCTACTTCTTCCATGCGTGATGTGTC-3' (SEQ ID No: 4).

Then, utilizing XhoI and KpnI sites, the amplified fragment was inserted into a pTagRFP-C1 vector. The inserted DNA fragment was introduced into a pEGFP-C1 (Clontech) vector to also construct an Hsp90-EGFP expression vector. These expression units were also introduced into the ViraPower Adenoviral Expression System (Thermo Fisher) in accordance with the manufacturer's instruction manuals.

EGFP-Rab18Q67L (Addgene plasmid #49596; https://www.addgene.org/49596; RRID:Addgene_49596), EGFP-Rab18S22N (Addgene plasmid #49597; https://www.addgene.org/49597; RRID:Addgene_49597), and EGFP-Rab18 (Addgene plasmid #49550; https://www.addgene.org/49550; RRID: Addgene_49550) expression vectors were kindly provided from Mr. Marci Scidmore (Huang et al., Cell Microbiol 12, 1292-1307, 2010).

As for the expression of GST-tagged Rab18 proteins in E. coli, the Rab18 fragment of each plasmid was amplified by PCR using the following primers, and the amplified fragment was then inserted into the EcoRI-NotI site of a pGEX-4T-3 vector (Cytiva Life Sciences):
5'-TCCGAATTCCATGGACGAGGACGTGCTGACCACTCTG-3' (SEQ ID No: 5), and
5'-AATGCGGCCGCCTATAGCACAGAGCAGTAACCGCCGCAGGCG-3' (SEQ ID No: 6).

### 1-4. Transfection and transduction

The expression vectors were introduced into cells, either by transfection of plasmids using Lipofectamine LTX-Plus (Thermo Fisher), or by transduction of adenoviral vectors.

### 1-5. Inhibitors

CID1067700 (#SML0545-5MG) was purchased from Sigma-Aldrich, was solubilized at 40 mM in DMSO, and was added to cells to a final concentration of 40 µM, followed by performing culture for 24 hours. GW4869 (#13127) was purchased from Cayman Chemical, was solubilized at 5 mM in DMSO, and was added to cells to a final concentration of 1.25 µM, followed by performing culture for 24 hours. TAS116 (#HY-15785) was purchased from MedChemExpress, was solubilized at 1 mM in DMSO and was added to cells to a final concentration of 0.5 µM, followed by performing culture for 48 hours.

### 1-6. Isolation of extracellular vesicles (EVs)

Isolation of extracellular vesicles from human mesenchymal stem cells was performed in accordance with MISEV2018 guidelines (Thery et al., Minimal information for studies of extracellular vesicles 2018 (MISEV2018): a position statement of the International Society for Extracellular Vesicles and update of the MISEV2014 guidelines. J Extracell Vesicles 7, 1535750 (2018)).

Human mesenchymal stem cells were plated on three 10-cm cell culture dishes at a density of 5,000 to 6,000 cells/cm². Six days after initiation of the culture, per dish, the medium was replaced with the EV Production Basal Medium (FUJIFILM-Wako, #053-09451 and # 298-84001), to which 10 mL of supplements had been added (wherein a new medium to be replaced comprised an additive compound, as appropriate). After 24 hours of culture under conditions of 5% CO₂ and 37°C, the medium was recovered, and was then centrifuged at 2,000 x g for 30 minutes at 4°C to remove the cell debris. Using a Millipore Amicon Ultra 30,000 MWCO filter (Pall, #MAP030C37), the resultant was centrifuged at 4,000 x g for 60 minutes at 4°C, so that it was condensed to 1 ml. To the condensed culture supernatant, 500 µL of Total Exosome Isolation Reagent (Invitrogen, #4478359) was added, and the obtained mixture was then incubated overnight, while mixing it by inverting. The solution that had been mixed overnight was centrifuged at 10,000 x g for at least 1 hour, and the extracellular vesicles were harvested as a precipitate, which was then resuspended in 30 µL of PBS. The obtained extracellular vesicles were preserved at 4°C and were then used in experiments within 1 week.

### 1-7. Nanoparticle tracking analysis (NTA)

The above-described culture supernatant was diluted with PBS, and was then analyzed using Zetaview Nanoparticle Tracking Analysis Instrument (Particle Metrix, Germany). The collected data were analyzed and diagrammatized using Prism software (Graphpad, ver. 7 and 9.3.1).

### 1-8. Observation by transmission electron microscopy

Observation by transmission electron microscopy (TEM) was performed according to previously reported standard methods (Nonaka et al., Cell 95, 829-837, 1998; Takei et al., The Journal of cell biology 131, 1789-1800, 1995). A precipitate of extracellular vesicles was fixed in Half Kalnovsky fixative at 37°C for 10 min and then at 4°C overnight, followed by post-fixation with 1% OsO₄ (in 0.1 M cacodylate buffer (pH 7.4)) on ice for 1.5 hours. Thereafter, the sample was subjected to electron staining with 1% uranyl acetate for 1 hour, and was then dehydrated using a graded ethanol series. Finally, the dehydrated extracellular vesicle sample was embedded in Quetol-812 (Nisshin EM), was then cut into ultrathin sections, was then electron-stained with uranyl acetate and lead citrate, and was then observed using a JEM2000 microscope at 100 keV, or using a JEM-1400Flash microscope at 80 keV.

### 1-9. In vitro extracellular vesicle release imaging

In order to monitor the Brownian motion of Shh extracellular particles, NIH3T3 cells were plated at 1 × 10⁴ cells/well onto an 8-well LabTek II Chamber #1.5 German Coverglass System pre-coated with poly-L-lysine. On the 3rd day of the culture, an adenoviral Shh-N-EGFP expression vector was introduced into the NIH3T3 cells. The medium was replaced with a complete medium that contained or did not contain an additive compound on the 4th day of the culture. Twenty-four hours after the medium replacement, time-lapse imaging was performed using a spinning disc microscope (Yokogawa/ZEISS) according to the previous report (Tanaka et al., Neuron 90, 1215-1229, 2016) under conditions of 5% CO₂ and 37°C at intervals of 5 seconds. Shh extracellular vesicles with Brownian motion were identified using Imaris 8 software (Bitplane) and were then quantified.

### 1-10. Angiogenesis assay

Angiogenesis assays were basically performed according to protocols provided by Lonza Corporation (Fujio et al., Journal of tissue engineering and regenerative medicine 11, 2116-2126, 2017). That is, an 8-well glass-bottomed chamber (Thermo Scientific Nunc, Denmark) was pre-coated with cold Matrigel (Corning, #356231) at 250 µl/well, and was then incubated at room temperature for 10 minutes, and then, under conditions of 5% CO₂ and 37°C for at least 30 minutes.

HUVEC cells that had been culture for 7 days were passaged at a density of 65,000 to 80,000 cells/cm² per well. Purified, fresh extracellular vesicles were added to each well, and the HUVEC cells were then imaged using an LSM780 confocal microscope (ZEISS) under conditions of5% CO₂ and 37°C, for at least 4 hours at multiple positions. As for extracellular vesicles, the total amount of extracellular vesicles collected from half the amount of the culture supernatant in a 10-cm dish was added to 1 cm² of the well. The data were collected using a Plan-Apochromat 10x/0.45 M27 lens by 488 nm laser light at a differential interference contrast microscopy mode. Thereafter, the collected data were analyzed using ImageJ software and were quantified for the number of formed blood vessels, the number of branching points of blood vessels, and the total tube length of blood vessels (Fujio et al., Journal of tissue engineering and regenerative medicine 11, 2116-2126, 2017; Lin et al., Frontiers in pharmacology 10, 1273, 2019).

### 1-11. GST pulldown assay

GST pulldown assays were basically performed according to the previous report (Niwa et al., Nature cell biology 10, 1269-1279, 2008). As for E. coli harvesting, B-PER Bacterial Cell Lysis Reagent (Thermo Fisher Scientific) was used.

### 1-12. Immunoblotting

Immunoblotting was performed according to the previous report (Yin et al., Neuron 70, 310-325, 2011). As for sample preparation, purified extracellular vesicles were dissolved in 1/3 of 4 x Laemmli's sample buffer, and were heat-treated at 98°C for 1 to 2 minutes. Thereafter, the sample was loaded on SDS-PAGE, were then transcribed onto a PVDF membrane (Immobilon-P, EMD Millipore). The transcribed PVDF membrane was incubated with primary and HRP-conjugated secondary antibodies diluted with Can Get Signal Solutions 1 and 2, and was then subjected to ECL (GE Healthcare Life Sciences, USA) procedures, so that bands recognized by the antibodies were visualized. The bands were detected using an ImageQuant LAS 4000 mini system (GE Healthcare Life Sciences, USA) and were then quantified using ImageJ software. Alternatively, the membrane was incubated with an alkaline phosphatase-conjugated secondary antibody (Cappel) according to the previous report (Kondo et al., J Cell Biol 125, 1095-1107), and color was developed using BCIP and NBT (Roche). The protein molecular weight marker was purchased from Bio-Rad (Herculus, CA,

### USA).

### 1-13. Fluorescence microscope

As for light microscopic observation, cells were plated on a 35-mm glass-bottomed dish (Matsunami, Japan, #D11130H) that had been pre-coated with poly-L-lysine (1:1,000, Sigma, #P4707) for 1 hour. Immunofluorescence labelling was performed using the antibodies described above. Briefly, cells were incubated with the primary antibody overnight at 4°C, and with the secondary antibody for 1 hour, followed by washing with PBS three times for 5 minutes for each washing. Thereafter, as mentioned above, using a spinning disc confocal microscope (Yokogawa/ZEISS) or an LSM780 confocal microscope equipped with Airyscan, the cells were observed. Three dimensional reconstruction of the cells was performed using Imaris 8 software (Bitplane).

### 2. Results

### 2-1. Rab Inhibitor CID1067700 facilitates Shh-EV release from human mesenchymal stem cells (hMSCs).

In order to investigate whether the Rab inhibitor CID1067700 (2-(benzoylcarbamothioylamino)-5,5-dimethyl-4,7-dihydrothieno[2,3-c]pyran-3- carboxylic acid or ML282) (hereinafter also referred to as "CID") can modulate the release of extracellular vesicles from human mesenchymal stem cells, an extracellular vesicle fraction was isolated from a culture supernatant (Figure 1A). The cells were treated with 40 µM CID1067700 or DMSO for 24 hours. Each culture supernatant was centrifuged to remove impurities, and the resultant was then mixed with Exosome Isolation Reagent (Invitrogen) to precipitate the extracellular vesicles. The extracellular vesicle fraction was subjected to immunoblotting using antibodies against Shh, TSG101, and Hsp90 (Figures 1B and 1C). Although the amounts of the general exosome markers TSG101 and Hsp90 were largely unaltered, the amount of Shh in the extracellular vesicles released from the cells treated with CID was significantly elevated, compared to that in the cells treated with DMSO. These results suggested a specific and significant increase in the production of Shh-containing extracellular vesicles.

### 2-2. CID1067700 augments angiogenesis activity of extracellular vesicles derived from human mesenchymal stem cells.

As a bioassay for elucidating the functions of extracellular vesicles derived from human mesenchymal stem cells stimulated with CID1067700, the influence of the CID1067700-treated extracellular vesicles on the in vitro angiogenesis of HUVEC cells was examined. HUVEC cells (6.5 to 8 × 10⁴) were mixed with extracellular vesicles isolated from a culture supernatant (10-cm plate) of human mesenchymal stem cells, and the obtained mixture was then plated on Matrigel-coated chamber slides. Ninety minutes after the plating, the levels of angiogenesis were compared (Figures 2A and 2B). As a result, when the HUVEC cells were treated with the extracellular vesicles derived from human mesenchymal stem cells, which had been stimulated with CID1067700, and when the number of blood vessels (Figure 2C), the number of branching points (Figure 2D), and the total tube length of blood vessels (Figure 2E) were then quantified, it became clear that angiogenesis was promoted by approximately 4 to 5 times that of the control. These results suggest that angiogenesis activity was significantly increased by extracellular vesicles derived from human mesenchymal stem cells treated with CID1067700.

### 2-3. CID1067700 facilitates production of extracellular vesicles with large particle diameter.

In order to investigate the influence of CID1067700 on the form of extracellular vesicles, nanoparticle tracking analysis (NTA) and transmission electron microscopic observation (TEM) were performed.

First, human mesenchymal stem cells were cultured for 24 hours in a basic medium for EV production (FUJIFILM-Wako) supplemented with supplements in the presence or absence of CID1067700, and NTA was performed using the culture supernatant after removing impurities by centrifugation (Figure 3). Using Zeta View system capable of counting extracellular vesicles with a large particle diameter such as approximately 1 µm, the measurement was performed at the "highest" mode. As a result, the sample derived from human mesenchymal stem cells treated with DMSO alone and the sample derived from human mesenchymal stem cells treated with CID1067700 contained almost the same amount of extracellular vesicles with a diameter of 100 to 800 nm per ml (the sample treated with DMSO alone: 4.4 ± 0.4 × 10⁸ particles/ml; the sample treated with CID1067700: 3.2 ± 1.9 × 10⁸ particles/ml, p ≥ 0.05, Welch's t test, n = 4) (Figure 3H). However, only in the culture supernatant of cells treated with CID1067700, extracellular vesicles with a diameter of larger than 800 nm were contained at a concentration of 6.6 ± 2.2 × 10⁷ particles/ml (p < 0.05, Welch's t test, n = 4; Figure 3I). These results indicate that CID1067700 specifically promotes production of large extracellular vesicles from human mesenchymal stem cells.

Next, the extracellular vesicle pellets were observed using TEM (Figure 4). In addition to conventionally known small (approximately 100 nm in diameter) exosome-like particles, particles with a low electron density (bright) and a diameter of larger than 800 nm were contained in the extracellular vesicle fraction derived from human mesenchymal stem cells treated with CID1067700, and the particles tended to be surrounded with a lipid double layer (Figures 4A and 4B). From the histogram of particle sizes larger than 100 nm, it became clear that the number of large-size extracellular vesicles was significantly increased by the CID1067700 treatment (Figures 4C and 4D). The results of the aforementioned electron microscopic observations also show that CID1067700 specifically promotes production of large extracellular vesicles derived from human stem cells.

### 2-4. CID1067700 increases production of Shh-EVs via Hsp90 and nSMase2 pathway.

Extracellular vesicles with a large particle diameter, which contain Shh, were visualized by live cell imaging in ShhN-EGFP transduced NIH3T3 fibroblasts treated with CID1067700 (Figure 5). As shown in Figure 5A, it was frequently observed that bright particles approximately 1 µm in size move randomly in the medium above the surface of the cultured cells.

These particles were automatically tracked and were identified as extracellular vesicles released from cells under drug (CID1067700, GW4869, or TSA116) addition conditions (Figures 5B to 5I). From the results of the live cell imaging, it was confirmed that the number of Shh-EVs was significantly increased after 24 hours of treatment with CID1067700, compared to the control (DMSO treatment) (Figure 5J). However, this increase in the released amount of Shh-EVs due to the CID1067700 treatment disappeared, when the cells were treated with 5 µM TSA116 (Hsp90 inhibitor) for 48 hours or with 1.25 µM GW4869 (nSMase2 inhibitor) for 24 hours (Figure 5J). These results suggest that Hsp90 and nSMase2 function as factors involved in the pathway of production of extracellular vesicles promoted by CID1067700.

### 2-5. CID1067700 promotes synthesis of ceramide via nSMase2.

In order to investigate whether nSMase2 activity is influenced by the CID1067700 treatment, NIH3T3 cells were treated with CID1067700 and/or GW4869, and immunostaining of ceramide was then performed (Figure 6). As a result, the expression level of ceramide in the cytoplasm was significantly increased after 24 hours of treatment with CID1067700, compared to treatment with DMSO alone (Figures 6B and 6D). Since this increase in the expression level of ceramide caused by CID1067700 was significantly suppressed, when the cells were simultaneously treated with GW4869 (Figures 6C and 6D), it was suggested that CID1067700 promotes nSMase2 activity and ceramide synthesis, and that it promotes Shh-EV production.

### 2-6. Rab18-GDP and Rab18-GTP are co-localized to AP-1, separately.

In order to investigate how the dynamics of Shh and Hsp90 are spatially regulated by the nucleotide state of Rab18, EGFP-Rab18S22N (a mutant that mimics the state of GDP-bound Rab18) or EGFP-Rab18Q67L (a mutant that mimics the state of GTP-bound Rab18) were stably transduced into NIH3T3 cells (Figure 7 and Figure 8). It was shown that Rab18S22N tends to accumulate in the perinuclear region, whereas Rab18Q67L tends to distribute in spotted organelles and cytoneme-like extracellular protrusions in the cytoplasm.

In order to investigate the properties of these Rab18-positive organelles, these stably transduced cells were subjected to immunostaining for the adaptor proteins AP-1 and AP-3 was carried out (Figure 7). Extremely interestingly, AP-1 was co-localized in perinuclear Rab18-S22N-positive organelles and Rab18-Q67L-positive cytoplasmic spotted organelle (Figure 7A). However, AP-3 and Rab18 showed mutually different localizations (Figure 7B). Since AP-1 has been reported to be involved in sorting from the trans-Golgi network (TGN) to endosomes (Delevoye et al., J Cell Biol 187, 247-264, 2009; Nakagawa et al., Cell 103, 569-5 81, 2000; Schmidt et al., National Academy of Sciences of the United States of America 106, 15344-15349, 2009), it is considered that Rab 18-GDP may be involved in the biosynthesis of exosomes via endosomal MVB (multivesicular body) pathway.

### 2-7. Rab18-GDP increases accumulation of Shh and Hsp90 in perinuclear region.

Next, cells expressing a Rab18 mutant were transfected with a ShhN-tagRFP expression vector (Figure 8A) or an Hsp90-tagRFP expression vector (Figure 8B), and were observed using a dual-wavelength fluorescence microscope. Under the expression of Rab18S22N, Shh and Hsp90 were observed to accumulate in the Golgi region together with Rab18S22N. However, under the expression of Rab18Q67L, the fluorescent signals from Shh and Hsp90 were observed to be more intense and diffusely distributed in the cytoplasm.

The fluorescent signals were quantified, and as a result, it was confirmed that the changes in the distribution of Shh and Hsp90 under the expression of Rab18 in different nucleotide states were statistically significant (Figures 8C to 8F). These data suggest that Rab18-GDP recruits Shh and Hsp90 to perinuclear organelles.

Furthermore, these cells were transfected with a ShhN-tagRFP expression vector, and the number of red fluorescent extracellular vesicles (Shh-containing extracellular vesicles) was measured in the cells (Figure 12). As a result, the number of red fluorescent extracellular vesicles in the culture supernatant of the cells transfected with Rab18S22N-EGFP was significantly larger than the number of red fluorescent extracellular vesicles in the culture supernatant of the cells transfected with Rab18Q67L-EGFP. These results suggest that the GTPase cycle of Rab18 solely controls the percentage of Shh-EV production.

### 2-7. Hsp90 and nSMase are effectors of Rab18-GDP.

In order to investigate whether Rab18 binds to different EV regulatory proteins depending on the state of the bound nucleotide, a GST pulldown assay was performed. First, GST-Rab18S22N and GST-Rab18Q67L, which were expressed in E. coli, were each added to mouse brain lysates, and GST pulldown was performed. As a result, KIF5A, a kinesin-1 motor protein, preferentially bound to Rab18-GTP (Figure 9). In contrast, Hsp90 and nSMase2 preferentially bound to Rab18-GDP, and it has been suggested that Rab18-GDP functions to ensure that Hsp90 and nSMase2 function properly during the accumulation and maturation of Shh-EV precursor vesicles and the process of their release to the outside of cells.

### 2-8. CID1067700 promotes cloud-like secretion of Hsp90 and Shh.

The morphological characteristics of Shh-EVs mediated by Hsp90 secreted from CID1067700-treated cells were observed. Both Shh-tagRFP and Hsp90-EGFP were transduced into NIH3T3 cells, and the effects obtained by treating the cells with CID1067700 for 24 hours were compared. The cells were fixed the cells and were then photographed by z-stacks, and a three-dimensional model was then constructed. Extremely interestingly, it was observed that many large extracellular structures having a height of 20 to 40 µm, which contained Hsp90 and Shh, floated above the layer of cells treated with CID1067700 (Figure 10). Since these structures were observed after the medium was removed and the cells were fixed, they are thought to interact with the cells as intermediate structures in the process of extracellular vesicle release. These structures are composed of two types of structures having a similar height. One shows a strongly compressed fluorescent image with a diameter of about 10 µm (Figure 10E), and the other shows a cloud-like fluorescent image with a diameter of about 20 to 30 µm, which contained many Shh/Hsp90 double-positive extracellular vesicles (Figure 10). The former is thought to be a precursor of the latter, and to be in a state in which a Shh-EV covered with a polymer containing Hsp90 is still coated with a membrane. The cells with these extracellular structures were significantly increased by the CID1067700 treatment (Figure 10G).

Summarizing the data presented in the present Examples, it is suggested that Rab18-GDP incorporates Hsp90 and nSMase2 into perinuclear multivesicular bodies and thereby promotes the release of Shh-EVs having a large particle diameter to the outside of cells (Figure 11).

### Industrial Applicability

According to the present invention, it is possible to promote secretion of extracellular vesicles containing a large amount of sonic hedgehog from cells. Since sonic hedgehog is involved in the healing of various diseases (e.g., ischemia, etc.) through the augment of angiogenesis, the present invention is expected to be utilized in the medical field.

## Claims

1. A method for producing extracellular vesicles, comprising obtaining extracellular vesicles secreted from cells subjected to a treatment of inactivating Rab.

2. The method according to claim 1, which is **characterized in that** the cells express sonic hedgehog.

3. The method according to claim 1, which is **characterized in that** the cells are mesenchymal stem cells.

4. The method according to claim 1, wherein the treatment of inactivating Rab GTPase is to treat with a Rab GTPase inhibitor.

5. The method according to claim 1, wherein the treatment of inactivating Rab GTPase is to transfect of a Rab18-GDP mutant into the cells.

6. The method according to any one of claim 1 to claim 5, which is **characterized in that** the extracellular vesicles contain sonic hedgehog.

7. The method according to any one of claim 1 to claim 5, which is **characterized in that** the extracellular vesicle has a particle diameter of 800 nm or more and/or a volume of 10⁹ nm³ or more.

8. A cell expressing sonic hedgehog, which is subjected to a treatment of inactivating Rab GTPase.

9. The cell according to claim 8, which retains Hsp90 gene and nSMase2 gene in an expressible state.

10. An extracellular vesicle containing sonic hedgehog and having a particle diameter of 800 nm or more and/or a volume of 10⁹ nm³ or more.

11. A medicament or a pharmaceutical composition, comprising, as an active ingredient, the extracellular vesicle according to claim 10.

12. A method for screening for a substance that inactivates Rab GTPase, comprising the following steps (a), and (b1), (b2) or (b3):
(a) a step of allowing a candidate substance to come into contact with cells expressing sonic hedgehog, and
(b1) a step of measuring the particle diameter and/or volume of extracellular vesicles secreted from the cells allowed to come into contact with the candidate substance in the step (a),
(b2) a step of confirming the presence of absence of accumulation of sonic hedgehog or Hsp90 around the cell nuclei of the cells allowed to come into contact with the candidate substance in the step (a), or
(b3) a step of confirming that the amount of KIF5A binding to Rab18 is decreased in the cells allowed to come into contact with the candidate substance in the step (a), or the amount of Hsp90 or nSMase is increased therein.

13. The method according to claim 12, further comprising a step (c) of confirming whether the extracellular vesicles of the step (b1) contain sonic hedgehog.

14. A method for screening a substance that activates Rab GTPase, comprising the following steps (d), and (e1) or (e2):
(d) a step of allowing a candidate substance to come into contact with cells expressing sonic hedgehog, and
(e1) a step of measuring the particle diameter and/or volume of extracellular vesicles secreted from the cells allowed to come into contact with the candidate substance in the step (d),
(e2) a step of confirming that the expression level of sonic hedgehog or Hsp90 in the cytoplasm is increased and that the sonic hedgehog or the Hsp90 is diffusely present in the cytoplasm, or
(e3) a step of confirming that the amount of KIF5A binding to Rab18 is increased in the cells allowed to come into contact with the candidate substance in the step (d), or the amount of Hsp90 or nSMase is increased therein.

15. The method according to claim 14, further comprising a step (f) of confirming whether the extracellular vesicles of the step (e1) contain sonic hedgehog.
